# EUROPEAN PATENT SPECIFICATION

(11) **EP 0 466 100 B1**
(45) Date of publication and mention of the grant of the patent: **15.02.1995**
(21) Application number: 91111410.6
(22) Date of filing: 09.07.1991
(51) Int. Cl.: A61F 5/04, A61F 13/04, A61L 15/07

(54) **Sheet and article using the same for orthosis**
Blatt und Gegenstand selbige verwendend für Orthese
Feuille et article comprenant celle-ci pour orthèse

(30) Priority: 09.07.1990 JP 179633/90
(43) Date of publication of application: 15.01.1992
(73) Proprietor: UNITIKA LTD., Amagasaki-shi Hyogo (JP)
(72) Inventor: Unigame, Tukasa, c/o Unitika Ltd., Uji-shi, Kyoto (JP); Itoi, Eiichi, c/o Unitika Ltd., Uji-shi, Kyoto (JP)
(74) Representative: Hansen, Bernd, Dr. Dipl.-Chem.

(56) References cited:
- EP-A- 0 326 285
- WO-A-89/11259
- DE-U- 8 618 257
- US-A- 4 473 671
- US-A- 4 600 618
- US-A- 4 893 617

## Description

### FIELD OF THE INVENTION

The present invention relates to an article using a sheet for orthosis related to surgery or orthopedics (for example, spinal orthosis, upper extremity orthosis, and lower extremity orthosis, particularly short leg orthosis, long leg orthosis and knee orthosis) which have fixing properties, mobility, the ability to closely fit any affected body part and which have excellent durability.

### BACKGROUND OF THE INVENTION

Metals, leathers and fabrics have been used as an aid for orthosis but are disadvantageous in that they are heavy, have a poor appearance, easily gather rust or are liable to become dirty. With the recent advancements in high polymer technology, the use of thermoplastic resins has been extending to orthotic articles. Thermoplastic resin materials are lightweight, have good appearance, are resistant to rust or contamination and can be kept clean. Further, they are flexible and may be shaped by heating to some extent. In addition, they can be molded into a shape to accurately fit a positive mold, e.g., a plaster cast that is shaped to fit an affected body part.

Methods for producing orthotic articles made mainly of thermoplastic resins include 1) a vacuum-forming method comprising covering a heat-softened thermoplastic resin sheet over a positive mold in an oven and evacuating the gap between the mold and the sheet by suction to bring the sheet into tight contact with the mold; and 2) a manual-forming method in which the thermoplastic resin sheet is brought in tight contact with the mold by hand.

Therefore, the thermoplastic materials to be used in the present invention should not only satisfy the requirements for application as an orthotic article but should also be easy to handle in a heat-softened state. Taking these requirements into consideration, conventionally employed thermoplastic resins mainly include ultra-high-molecular-weight polyethylene, high-density polyethylene, and polypropylene, although the resins vary depending on the conditions of a patient and the site of application to the body.

Comparing conventional thermoplastic polyolefin resins and metals in mechanical characteristics, metals have high strength and excellent mobility around joints, whereas thermoplastic polyolefin resins, when applied to a site containing a joint, have poor strength, insufficient fixing properties, and insufficient mobility at the joint. For example, a shoehorn type short lower leg brace made of a thermoplastic resin which is used not only for walking but for activities in daily life is effective for correcting or preventing deformation of the ankle joint or preventing plantar flexion of the foot during walking. However, in movements needing dorsiflexion of the ankle joint as in squatting or going up and down stairs, the orthotic article is apt to deviate from the physical joint axis, causing discomfort, and to undergo breakage on repetition of the movement.

If the thickness of the resin sheet is increased to have increased strength, fixing properties on ankle joints, etc. may be improved, but the sheet loses mobility in the joint portion and the weight is increased which accentuates discomfort. In addition, the article becomes bulky across the instep, making ready-made shoes ill-fitting.

Further, since shoehorn type short lower leg braces made of thermoplastic resins do not take into account physiological inversion and eversion of the ankle joint, it is nearly impossible for the users of such braces to control movement when crossing a slope or swaying the trunk. That is, they have such problems as induction of involuntary movements, loss of residual functions, and occurrence of secondary disturbances of the knee joint.

Hence, there is a demand for a material which is soft and elastic for the purpose of obtaining a balance between the conflicting characteristics, i.e., fixing properties and mobility, and which is also easily formed.

US-A-4 473 671 reveals a thermoplastic casting material having a softening temperature of from 45°C to 85°C and capable of recrystallizing to form a rigid self supporting cast within 10 minutes of being softened comprising from 40% to 70% by weight of a crystalline polyurethane polymer and 30% to 60% of a filler.

### SUMMARY OF THE INVENTION

An object of the present invention is to provide an orthotic article using a resin sheet which is easily formed into orthotic articles having a balance between fixing properties and mobility, satisfactory applicability to an affected body part, and excellent durability.

It has now been found that a sheet of a thermoplastic polyurethane resin having specific physical properties exhibits elasticity and softness and that such a sheet is easily formed into an orthotic article satisfying conflicting performance properties of fixing and mobility.

The present invention provides an orthotic article which comprises a sheet of a thermoplastic polyurethane resin having a rebound resilience of from 30 to 80%, a Shore D hardness of from 30 to 60, an ultimate tensile strength of not less than 150 kg/cm², a tear strength of not less than 70 kg/cm, and a melting point of from 110 to 230°C as measured with a differential scanning calorimeter (DSC).

The above article for orthosis is obtained by forming a sheet of a thermoplastic polyurethane resin as defined above to fit an affected body part of a patient.

A preferred embodiment of the present invention provides an article for an ankle foot orthosis, and particularly the article which widely covers the body from the transitional part of triceps surae-tendon to the tiptoe and has a longitudinal open slit on the front from the leg part to the tiptoe and fixing belts across the slit.

### BRIEF DESCRIPTION OF THE DRAWINGS

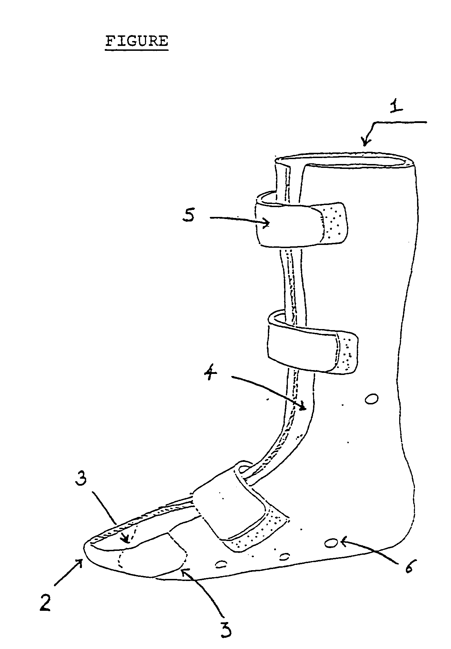

The Figure illustrates an article for ankle foot orthosis according to the present invention.

### DETAILED DESCRIPTION OF THE INVENTION

Rebound resilience, Shore D hardness, ultimate tensile strength, and tear strength as referred to in the present invention are measured according to the "rebound resilience test method", "hardness test method", "tensile test method", and "tear test method" of JIS K-7311-1987 (JIS as used herein refers to Japanese Industrial Standard), respectively. Melting point as referred to in the present invention is measured with a DSC manufactured by Perkin Elmer Co.

In the present invention, a sheet for producing orthotic articles, particularly an article for ankle/foot orthosis, is prepared from a thermoplastic polyurethane resin having specific physical properties, such as rebound resilience, Shore D hardness, ultimate tensile strength, tear strength, and melting point, and that exhibits excellent moldability and chemical and mechanical properties inherent in the above properties. The sheet can easily be formed into orthotic articles having an excellent balance berween fixing and mobility at the joints, etc., the ability to fit any affected body part and excellent durability.

The thermoplastic polyurethane resin which can be used in the present invention are polyurethane elastomers which are obtained from a high-molecular polyol, which constitutes a soft segment, a diisocyanate compound, which constitutes a hard segment, and a chain extender or a crosslinking agent. The type and proportions of these raw materials are selected as desired. The thermoplastic polyurethane resin basically comprises a rubbery soft segment and a crystalline hard segment.

Polyols constituting the soft segment include polyester polyols and polyether polyols.

Polyester polyols are generally obtained from general purpose OH-terminated glycol adipates, e.g., polyethylene adipate and polybutylene adipate. Polyester polyols are also obtained from polydiethylene adipate and polyhexane adipate which are liquid at room temperature. Polyester polyols obtained by using an aromatic acid (e.g., phthalic acid) as part of an acid component or a special dibasic acid (e.g., succinic acid) are also employable. In addition to these polyester polyols obtained from a glycol component and an acid component, polycaprolactones are widely employed.

Polypropylene glycol is widely used as a polyether polyol because it has low viscosity and is inexpensive. Where higher physical properties are demanded, polytetramethylene glycol can be used.

Diisocyanate compounds constituting the hard segment include tolylene diisocyanate, 4,4'-diphenylmethane diisocyanate, 1,5-naphthalene diisocyanate, 3,3'-tolidine diisocyanate, 1,6-hexamethylene diisocyanate, isophorone diisocyanate, xylene diisocyanate, p-phenylene diisocyanate, and cyclohexamethylene diisocyanate.

Chain extenders include aliphatic crosslinking agents such as monomolecular glycols, e.g., 1,4-butylene glycol; and aromatic chain extenders such as hydroquinone diethylol ether.

The kinds and ratios of the soft segment, hard segment, chain extender, crosslinking agent, and so on are appropriately selected so as to obtain a thermoplastic polyurethane resin having performance properties according to the end use.

The thermoplastic polyurethane resin of the present invention may be used, if desired, in combination with a minor proportion of other resins such as polyvinyl chloride resins, acrylonitrile-butadiene-styrene resins, polycarbonate resins, and polyacetal resins. In this case, the proportion of the thermoplastic polyurethane resin in the polymer blend is preferably at least 70% by weight.

Rebound resilience of the thermoplastic polyurethane resin is a factor in selecting resin materials, taking elasticity and softness into consideration for satisfying the conflicting requirements of fixing and mobility.

If the rebounsd resilience is less than 30%, the orthotic article is soft but poor in elasticity and fixing. If it exceeds 80%, a great force is needed to bend the material during use, imposing a great burden on the body part fitted with the article. Accordingly, the thermoplastic polyurethane resin should have a rebound resilience in the range of from 30 to 80%, and preferably from 35 to 75%, varying within that range depending on the site to be fitted with the article, the degree of deformation to be corrected, and the age, physical strength or sex of the patient.

Shore D hardness of the thermoplastic polyurethane resin should be in the range of from 30 to 60, and preferably from 35 to 55, varying within that range depending on the requisite degree of correction of deformation, local fixing or supporting, or weight bearing.

Ultimate tensile strength and tear strength of the thermoplastic polyurethane resin contribute to durability and should be selected according to the age, physical power or sex of the patient. In application even to infants who requires the least strength, the resin should have an ultimate tensile strength of not less than 150 kg/cm² and a tear strength of not less than 70 kg/cm. In practice, the ultimate tensile strength is preferably not more than 500 kg/cm², and the tear strength is preferably not more than 250 kg/cm.

Melting point of the thermoplastic polyurethane resin measured with a DSC is a guide for setting the temperature of an oven for softening the forming sheet molded from the thermoplastic polyurethane resin to be covered over a positive model, e.g., a plaster cast from a patient.

The lower the melting point, the easier it is produce an orthotic article. However, if the melting point is lower than 110°C, the heat distortion temperature or Vicat softening point become 100°C or lower, and the article may undergo plastic deformation or partial fluidization on heating when a patient takes a bath with the article on or when the article is sterilized or washed with hot water.

If the melting point is higher than 230°C, the resulting orthotic article has improved heat resistance, but workability and safety in the production of the articles are impaired.

Taking both heat resistance of the articles and production into consideration, a preferred melting point is in the range of from 150 to 190°C.

The orthotic articles prepared from the above-described thermoplastic polyurethane resin are applicable to the upper extremities or fingers when designed to have soft mechanical properties or to the spine when designed to have stiff mechanical properties. In particular, they are advantageously applicable to the ankle joint and surrounding areas for short leg orthosis.

The article for short leg orthosis according to the present invention preferably has the form illustrated in the Figure so that the excellent mechanical strength and elasticity of the thermoplastic polyurethane resin of the present invention can be used to their full advantage. The short leg orthotic article of the present invention has a basic structure which widely covers the body from the triceps surae-Achilles tendon transitional part to the tiptoe.

The height of top opening 1 can be adjusted to agree with the degree of ankle joint deformation or the patient's body weight. Tiptoe 2 is necessary for patients suffering from serious claw-toe, while the distal portion from the proximal metatarsophalangeal joint 3 may be cut off when the article is applied to a foot which is not seriously affected by claw-toe so that the patient may wear commercially available ready-made shoes. Correction of mild claw-toe or fixing and supporting of the toe may be achieved by the combined use of the short leg orthosis with shoes.

Longitudinal open slit 4 is provided on the front from the leg part to the tiptoe so as not to press the crest of the tibia and the extensor hallucis longus tendon. The width of slit 4 is chosen based on the desired fit and fixing properties for the affected body part while taking into consideration the volume and shape of the affected body part.

Fixing belts 5 are provided across the slit. Fixing belts 5 are usually made of planar fastening tape. Fixing belts 5 should be fitted at positions suitable for striking a balance between fixing and mobility. The number and positions of the belts are selected according to each patient.

Where the article covering a wide area of a foot lacks breathability, vent holes 6 may be provided at positions which do not substantially participate in the dynamic action during walking, and the size and number of the holes should be carefully considered.

The sheet being used in accordance with the present invention can usually be prepared by extrusion by means of a sheet extruder. Orthotic articles of the present invention can be produced by covering a heat-softened sheet on a positive model, etc. and bringing the sheet into close contact with the model by evacuating the air in the space between the sheet and the model by suction or by applying fluid pressure, manual pressure, or mechanical force. Alternatively, an extruded sheet which is still warm from the extrusion process may be formed (shaped) while soft into an article by blowing, pressing or vacuum forming before being cooled. It is also possible to directly mold the thermoplastic polyurethane resin into an article by injection molding, etc.

The thickness of the sheet of the orthotic article is not particularly limited, and usually ranges from 1 to 5 mm, and preferably from 2 to 4 mm, for practical use.

The above-described thermoplastic polyurethane resin contains inorganic fillers for improving hardness, formability and physical properties and for increasing the softening speed.

Specific but non-limiting examples of suitable inorganic fillers include silicone compounds, e.g., white carbon; alkaline earth metal sulfates, e.g., barium sulfate and calcium sulfate; alkaline earth metal carbonates, e.g., calcium carbonate and basic magnesium carbonate; polyvalent metal silicates, e.g., aluminum silicate, zinc silicate, calcium silicate, magnesium silicate, and zirconium silicate; polyvalent metal oxides, e.g., strontium oxide, magnesium oxide, calcium oxide, zinc oxide, aluminum oxide, titanium oxide, zirconium oxide, and silica; polyvalent metal hydroxides, e.g., magnesium hydroxide, strontium hydroxide, calcium hydroxide, zinc hydroxide, aluminum hydroxide, and zirconium hydroxide; clay minerals, e.g., kaolin, talc, mica, pyrophylite, montmorillonite, and sericite; lithopone comprised of barium sulfate and zinc sulfate; cristobalite; cellulose; glass fiber; glass powder; carbon black; graphite; copper powder; and aluminum powder. Of these, fine particles of titanium oxide, carbon black, and copper powder are most commonly employed.

These inorganic fillers are used in an amount of less than 30% by weight based on the sheet. If the proportion of the inorganic filler exceeds 30% by weight, the sheet undergoes a change in physical properties, such as an increase in hardness, resulting in brittleness.

The thermoplastic polyurethane resin may further contain other additives for various purposes, such as pigments, e.g., titanium white, red iron oxide, toluidine red, and benzidine yellow; colorants, e.g., edible red No. 3, edible green No. 3, and edible blue No. 1; mercury compounds, e.g., Thimerosal and Mercurochrome; silver compounds, e.g., silver nitrate and silver protein; chlorine compound preparations, e.g., chloramine, potassium chlorate, and chlorohexidine sulfate; iodine compound preparations, e.g., iodine and iodinated glycerol; and bactericides or antibiotics, e.g., hydroxyapatite-on-copper and phosphate-on-silver which are salts with small amount of metalic carrier.

The above-mentioned additives such as inorganic fillers, pigments, colorants, bactericides and antibiotics can be incorporated into the resin by means of a temperature-controlled mixer, kneader, extruder, etc. These additives may also be coated or printed on the surface of the polyurethane resin sheet.

The present invention is now illustrated in greater detail by way of Examples, but it should be understood that the present invention is not deemed to be limited thereto.

### EXAMPLE 1

Pellets of a thermoplastic polyurethane elastomer having a rebound resilience of 40%, a Shore D hardness of 49, an ultimate tensile strength of 530 kg/cm², a tear strength of 120 kg/cm, and a DSC melting point of 168°C ("PELLETHANE 2102-90 AE" produced by Dow Chemical Co., Japan) were extruded in a sheet extruder to obtain a sheet measuring 500 mm x 500 mm x 3 mm. The sheet was softened by heating in an oven at 180°C for 10 minutes and subjected to vacuum forming on a positive plaster model of a short leg to obtain a formed article having a wall thickness of from 2 to 2.5 mm. Similar formed articles could be obtained with satisfactory reproducibility, by conducting the above process.

The formed article was modified and trimmed, and the resulting short leg brace was fitted to a patient suffering from slight spastic pes equinovarus. As a result, shuffling of the toe was reduced by the moderate fixing by the brace to improve gait and to obtain stability in gait while assuring mobility in dorsiflexion of the ankle joint to allow the patient to squat with ease. Further, the patient could put on his shoes with the brace on and therefore could go out of doors.

### EXAMPLE 2

Pellets of a thermoplastic polyurethane elastomer having a rebound resilience of 55%, a Shore D hardness of 45, an ultimate tensile strength of 490 kg/cm², a tear strength of 90 kg/cm, and a DSC melting point of 164°C ("PELLETHANE 2102-80 AE" produced by Dow Chemical Co., Japan) were extruded in a sheet extruder to obtain a sheet of 500 mm x 500 mm x 3 mm. The sheet was softened by heating in an oven at 180°C for 8 minutes and subjected to vacuum forming on a positive plaster model of a short leg to obtain a formed article having a wall thickness of from 2 to 2.5 mm, with good reproducibility. After modification and trimming, the resulting short leg brace was fitted to an infant patient suffering from mild pes equinovarus. This short leg brace had increased softness as compared with that of Example 1, and thus was comfortable during use. Since the fixing by the brace was sufficient, shuffling of the toe was reduced, the patient could swing his leg with more ease, and flexion of the knee joint increased, thereby improving gait.

### COMPARATIVE EXAMPLE 1

Pellets of a thermoplastic polyurethane elastomer having a rebound resilience of 65%, a Shore D hardness of 20, an ultimate tensile strength of 106 kg/cm², a tear strength of 62 kg/cm, and a DSC melting point of 127°C ("PELLETHANE 2352-70 A" produced by Dow Chemical Co., Japan) were extruded in a sheet extruder to obtain a sheet of 500 mm x 500 mm x 3 mm. The sheet was softened by heating in an oven at 180°C for 10 minutes and subjected to vacuum forming on a positive plaster model taken on a short leg to obtain a formed article having a wall thickness of from 2 to 2.5 mm. After modification and trimming, the resulting short leg brace was fitted to a patient suffering from slight pes equinovarus. As a result, no improvement in shuffling of the toe was obtained due to low elasticity of the brace, and the gait was unsteady.

While the invention has been described in detail and with reference to specific examples thereof, it will be apparent to one skilled in the art that various changes and modifications can be made therein without departing from the scope thereof.

## Claims

1. An article for orthosis which is formed so as to fit an area of a body which comprises a sheet of thermoplastic polyurethane resin having a rebound resilience of from 30 to 80%, a Shore D hardness of from 30 to 60, an ultimate tensile strength of not less than 150 kg/cm², a tear strength of not less than 70 kg/cm, and a melting point of from 110 to 230°C as measured with a differential scanning calorimeter, and containing inorganic fillers in an amount of less than 30% by weight.

2. An article for orthosis as in claim 1, wherein the rebound resilience is from 35 to 75%.

3. An article for orthosis as in claim 1, wherein the Shore D hardness is from 35 to 55.

4. An article for orthosis as in claim 1, wherein the ultimate tensile strength is from 150 kg/cm² to 500 kg/cm².

5. An article for orthosis as in claim 1, wherein the tear strength is from 70 kg/cm to 250 kg/cm.

6. An article for orthosis as in claim 1, wherein the melting point is from 150 to 190°C as measured with the differential scanning calorimeter.

7. An article for orthosis as in claim 1, wherein the sheet has a thickness of from 1 to 5 mm.

8. An article for orthosis as in claim 7, wherein the sheet has a thickness of from 2 to 4 mm.

9. An article for orthosis as in claim 1, wherein the article is a short leg brace.

10. An article for orthosis as in claim 9, wherein the short leg brace widely covers from the transitional part of triceps surae-Achilles tendon to the tiptoe and has a longitudinal open slit on the front thereof from the leg part to the tiptoe and fixing belts across the slit.

## Patentansprüche

1. Gegenstand für die Orthese, der zur Anpassung an einen Teil eines Körpers geformt ist und der eine Folie aus thermoplastischem Polyurethanharz umfaßt, die eine Rückprallelastizität von 30 bis 80 %, eine Shore-D-Härte von 30 bis 60, eine Zugfestigkeit von nicht weniger als 150 kg/cm², eine Weiterreißfestigkeit von nicht weniger als 70 kg/cm und einen Schmelzpunkt von 110 bis 230°C, gemessen mit einem Kalorimeter mit Differentialabtastung, hat und anorganische Füllstoffe in einer Menge von weniger als 30 Gew.% enthält.

2. Gegenstand für die Orthese nach Anspruch 1, bei dem die Rückprallelastizität 35 bis 75 % ist.

3. Gegenstand für die Orthese nach Anspruch 1, bei dem die Shore-D-Härte 35 bis 55 ist.

4. Gegenstand für die Orthese nach Anspruch 1, bei dem die Zugfestigkeit 150 kg/cm² bis 500 kg/cm² ist.

5. Gegenstand für die Orthese nach Anspruch 1, bei dem die Weiterreißfestigkeit 70 kg/cm bis 250 kg/cm ist.

6. Gegenstand für die Orthese nach Anspruch 1, bei dem der Schmelzpunkt, gemessen mit dem Kalorimeter mit Differentialabtastung, 150 bis 190°C ist.

7. Gegenstand für die Orthese nach Anspruch 1, bei dem die Folie eine Dicke von 1 bis 5 mm hat.

8. Gegenstand für die Orthese nach Anspruch 7, bei dem die Folie eine Dicke von 2 bis 4 mm hat.

9. Gegenstand für die Orthese nach Anspruch 1, wobei der Gegenstand ein kurzes Bein-Stützkorsett ist.

10. Gegenstand für die Orthese nach Anspruch 9, bei dem das kurze Bein-Stützkorsett den Zwischenteil Wadentrizeps-Achillessehne bis zu der Zehenspitze weitgehend bedeckt und einen offenen Längsschlitz an seiner Vorderseite vom Beinteil bis zu der Zehenspitze und Befestigungsgurte quer über den Schlitz aufweist.

## Revendications

1. Article pour orthèse qui est formé de manière à s'adapter à une zone d'un corps, qui comprend une feuille d'une résine de polyuréthanne thermoplastique ayant une élasticité de rebondissement de 30 à 80 %, une dureté Shore D de 30 à 60, une résistance à la traction d'au moins 150 kg/cm², une résistance au déchirement d'au moins 70 kg/cm et un point de fusion, mesuré avec un calorimètre différentiel à balayage, de 110 à 230°C, et contenant des charges inorganiques en une quantité inférieure à 30 % en masse.

2. Article pour orthèse selon la revendication 1, dans lequel l'élasticité de rebondissement est de 35 à 75 %.

3. Article pour orthèse selon la revendication 1, dans lequel la dureté Shore D est de 35 à 55.

4. Article pour orthèse selon la revendication 1, dans lequel la résistance à la traction est de 150 kg/cm² à 500 kg/cm².

5. Article pour orthèse selon la revendication 1, dans lequel la résistance au déchirement est de 70 kg/cm à 250 kg/cm.

6. Article pour orthèse selon la revendication 1, dans lequel le point de fusion, mesuré au moyen d'un calorimètre différentiel à balayage, est de 150 à 190°C.

7. Article pour orthèse selon la revendication 1, dans lequel la feuille a une épaisseur de 1 à 5 mm.

8. Article pour orthèse selon la revendication 7, dans lequel la feuille a une épaisseur de 2 à 4 mm.

9. Article pour orthèse selon la revendication 1, dans lequel l'article est un appareil orthopédique de jambe courte.

10. Article pour orthèse selon la revendication 9, dans lequel l'appareil orthopédique de jambe courte recouvre largement depuis la partie de transition du triceps sural-tendon d'Achille jusqu'à la pointe du pied et a une fente ouverte longitudinale sur le devant depuis la partie jambe jusqu'à la pointe du pied et des sangles de fixation en travers de la fente.
